# EUROPEAN PATENT APPLICATION

(11) **EP 1 223 165 A1**
(43) Date of publication of application: **17.07.2002**
(21) Application number: 01100893.5
(22) Date of filing: 16.01.2001
(51) Int. Cl.: C07D 231/44, A01N 43/56

(54) **Processes for the preparation of pesticidal compound**

(71) Applicant: AVENTIS CROPSCIENCE S.A., 69009 Lyon (FR)
(72) Inventor: Rousseau, Jean-François, 69003 Lyon (FR); Buforn, Albert, 69800 Saint Priest (FR)
(74) Representative: Mérigeault, Shona

(57) **Abstract**

Processes for the Preparation of Pesticidal Compound a process (A) for the preparation of a compound of formula (I) 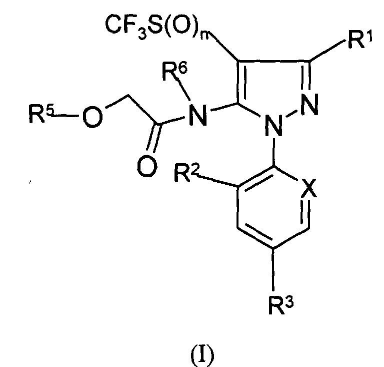 wherein:R¹ is CN or CSNH₂; X is N or CR⁴; R² and R⁴ are, each, independently hydrogen or chlorine; R³ is halogen, haloalkyl, haloalkoxy or -SF₅; R⁵ and R⁶ are each independently an alkyl group; and n is 0, 1 or 2;
which process comprises (a) a first step of reacting a compound of formula (II): 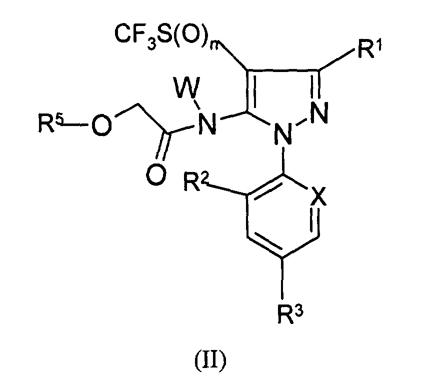 wherein the various symbols are as defined above and W is H , with an inorganic metal salt or an organic base to produce an intermediate compound, (b) a second step of reacting the intermediate compound with an alkylating agent of formula (III):

R⁶-Y (III)

wherein R⁶ is as defined above and Y is a leaving group. The intermediate compounds are also claimed as novel compounds.

## Description

The present invention relates to a process for the preparation of substituted pyrazoles and to their use as pesticidal compound.

Pyrazoles such as 5-Amino-1-aryl-3-cyanopyrazole compounds and derivatives thereof, for example Fipronil, form an important class of insecticides. Certain substituted 5-N-alkyl-N-alkoxyacetylamino-1-aryl-3-cyanopyrazole compounds have valuable pesticidal properties.

US Patent No. 4931461 discloses substituted 5-methylamino-1-aryl pyrazoles and their use as pest-combating agents. These substituted compounds may be prepared in various ways but in particular it has been found that the compounds may be prepared by reacting the pyrazole with an alkylating agent. This preparation method, whilst being effective, produces by-products that must be isolated from the desired pesticidal compound.

We have found an alternative route to the production of the aforementioned compounds which reduces and substantially eliminates the presence of by-products, thus avoiding the need to purify the final product.

Accordingly, the present invention provides a process (A) for the preparation of a compound of formula (I) 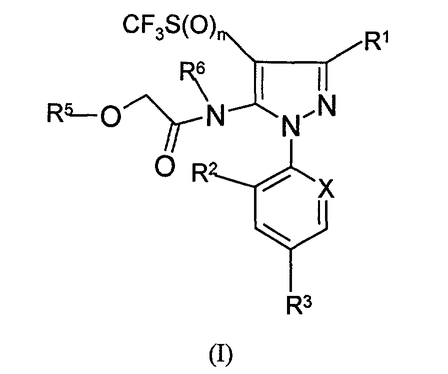 wherein:
R¹ is CN or CSNH₂;
X is N or CR⁴;
R² and R⁴ are, each, independently hydrogen or chlorine;
R³ is halogen, haloalkyl, haloalkoxy or -SF₅;
R⁵ and R⁶ are each independently an alkyl group; and
n is 0, 1 or 2;
which process comprises (a) a first step of reacting a compound of formula (II): 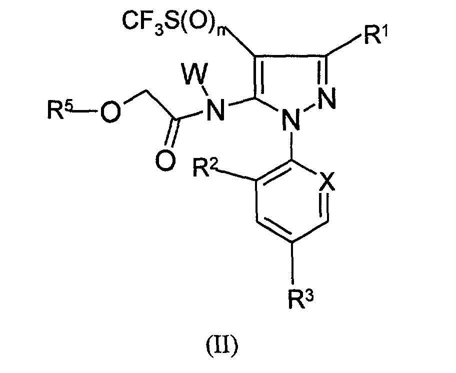 wherein the various symbols are as defined above and W is H , with an inorganic metal salt or an organic base to produce an intermediate compound, (b) a second step of reacting the intermediate compound with an alkylating agent of formula (III):

R⁶-Y (III)

wherein R⁶ is as defined above and Y is a leaving group.

The process of the present invention provides the advantage over the prior art in that there are no by-products produced during the reaction and that if desired, the intermediate compound may be isolated. Furthermore, it has been found that with this process, the intermediate compound is stable and may be isolated, if desired.

Furthermore, the intermediate compound obtained by the aforementioned process is a novel compound and hereby provides another aspect of the present invention. The process of the present invention comprises a first step of reacting a compound of General Formula (II) with an inorganic metal salt or an organic base. With regard to R³ of Compound II, this group may be halogen, haloalkyl, haloalkoxy or -SF₅. Where R³ is a haloalkyl. Suitable haloalkyls are halomethyls, especially trifluoromethyl. Where R³ is a haloalkoxy, suitable haloalkoxy groups include halomethoxy, in particular trifluoromethoxy. With regard to R⁵, this group is an alkyl group, for example methyl, ethyl or propyl, especially ethyl.

Preferably, the compound of General Formula (II) has the following representations:
R¹ is CN;
X is CR⁴;
R² and R⁴ are each chlorine,
R³ is trifluoromethyl;
R⁵ is ethyl,
W is H; and
n is 1.

The inorganic metal salt is suitably a Group I or II metal salt selected from cesium, potassium, sodium, calcium and magnesium. Preferably, the metal salt is a potassium or sodium metal salt. The salt may be in the aqueous or solid form and may suitably be a hydroxide, a carbonate or a hydrogen carbonate. The preferred salt for use in the process of the present invention is potassium carbonate or potassium hydroxide.

The organic base is suitably an amine, for example triethyl amine, pyridine and the like.

The compound of General Formula (II) is reacted with the metal salt or the organic base in a ratio of at least 1 equivalent, preferably 2 equivalents.

The first step may be carried out in the presence of a solvent, preferably a polar organic solvent which may be selected from ethers such as tetrahydrofuran, t-butylmethylether, dioxan, di-isopropyl ether and di-butyl ether; halogenated aromatis or aliphatic hydrocarbons such as dichloromethane, 1,2dichloroethane and monochlorobenzene; polar nitriles and amides such as acetonitrile, N,N-dimethylformamide and N-methyl pyrrolidinone or a mixture thereof. The preferred solvent is acetonitrile, N,N-dimethylformamide and N-methyl pyrrolidinone. There may also be present a non -polar solvent such as toluene. The solvent is suitable present in excess.

The intermediate product obtained is a novel product and herewith provides another aspect of the present invention. In particular when the compound of General Formula II is reacted with potassium carbonate to generate the potassium salt or with triethyl amine to produce the amine salt.

The intermediate compound, is then reacted with an alkylating agent of General Formula (III). The alkylating agent may be selected from alkyl sulphonates, alkyl halides or alkyl sulphates. The alkyl group may be methyl, ethyl, propyl or isopropyl. Where the alkylating agent is a halide, preferably the agent is chloride, bromide or iodide. Where the alkylating agent is a sulphonate, it is preferred to use di-methyl sulphonate or methyl aryl sulphonate. Where the alkylating agent is a sulphate, the preferred sulphate is di-methyl sulphate. The preferred alkylating agent is methyl bromide, methyl iodide or salts thereof and di-methyl sulphate.

The ratio of alkylating agent to the intermediate metal salt is suitably up to 10 equivalents, preferably from 1 to 20, especially from 5 to 10 equivalents.

The second step of the process may also be carried out in the presence of a base. Bases suitable for use in this second step include alkali metal hydrides, for example sodium hydride; alkali metal carbonates such as potassium carbonate or sodium carbonate or hydrogen carbonates; alkali metal alkoxides for example sodium methoxide; alkali metal hydroxides, for example sodium hydroxide and potassium hydroxide. Alternatively, the second step may be carried out in the presence of an organic base such as pyridine or tri-ethylamine; or a quaternary ammonium salt such as benzyltriethylammonium halide, for example the chloride or bromide salt or salts of R₄NOH, R₄NOalkyl for example Bu₄NOH. The preferred base is potassium carbonate or potassium hydroxide.

The second step of the reaction also may be carried out in the presence of a solvent, preferably a polar organic solvent which may be selected from ethers such as tetrahydrofuran, t-butylmethylether, dioxan, di-isopropyl ether and di-butyl ether; halogenated aromatics or aliphatic hydrocarbons such as dichloromethane, 1,2dichloroethane and monochlorobenzene; polar nitriles and amides such as acetonitrile, N,N-dimethylformamide and N-methyl pyrrolidinone. The preferred solvent is acetonitrile, N,N-dimethylformamide and N-methyl pyrrolidinone. There may also be present a non -polar solvent such as toluene. The solvent is suitable present in excess.

The process may be carried out at a temperature of from reaction temperature 0°C to 150°C, preferably from 20°C to 90°C and at atmospheric or elevated pressure.
The process of the present invention is particularly preferred for the production of a compound according to General Formula (I) where:
R¹ is CN
X is CR₄
R² and R⁴ are each, chloride
R³ is trifluoromethyl,
R⁵ is ethyl
R⁶ is methyl; and
n is 1

The compounds of formula (II) may be obtained by a process (B), wherein a compound of formula (IV): 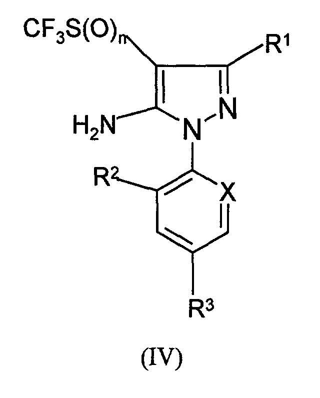 wherein the various symbols are as defined above, is reacted with an acylating agent of formula (V) or formula (VI): 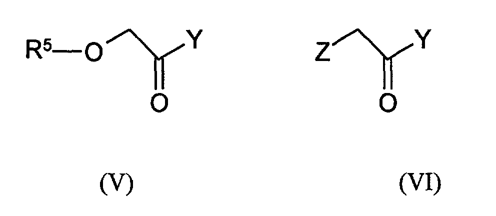 wherein R⁵ is as defined above and Y is a halide, especially chloride or bromide; alkoxy, anhydride, especially halide, e.g. chloride and Z is a halide, for example chloride, bromide and iodide.

The preferred compound of Formula (V) is when R⁵ is ethyl and Y is chloride and for Compound (VI), when Z is chloride and Y is chloride.

The process (B) is preferably carried out in the presence of a solvent, preferably a polar organic solvent which may be selected from ethers such as tetrahydrofuran, t-butylmethylether, dioxan, di-isopropyl ether and di-butyl ether; halogenated aromatic or aliphatic hydrocarbons such as dichloromethane, 1,2dichloroethane and monochlorobenzene; polar nitriles and amides such as acetonitrile, N,N-dimethylformamide and N-methyl pyrolidinone or a mixture thereof. The preferred solvent is acetonitrile, N,N-dimethylformamide and N-methyl pyrolidinone. There may also be present water or a non -polar solvent such as toluene. The solvent is suitable present in excess.

The process (B) is also preferably carried out in the presence of an organic or inorganic base. Bases suitable for use in this process include alkali metal hydrides, for example sodium hydride; alkali metal carbonates such as potassium carbonate or sodium carbonate or hydrogen carbonates; alkali metal alkoxides for example sodium methoxide; alkali metal hydroxides, for example sodium hydroxide and potassium hydroxide. Alternatively, the reaction may be carried out in the presence of an organic base such as pyridine or tri-ethylamine; or a quaternary ammonium salt such as benzyltriethylammonium halide, for example the chloride or bromide salt or salts of R₄NOH, R₄NOalkyl for example Bu₄NOH. The preferred base is potassium hydroxide, sodium hydroxide and tri-ethylamine. The reaction temperature is generally from minus 20°C to 150°C, preferably from 20°C to 90°C.

In a particular embodiment of the present invention, when compound (VI) is used to produce Compound II, and Z and Y are each chloride, this compound is reacted in the presence of a metal alkoxide, for example sodium ethoxide.

Compounds of formula (III), (IV) and (V) are known or may be prepared by known methods.

The present invention will now be illustrated by reference to the following examples:

### Example 1

Step 1: Preparation of the potassium salt of 1 -(2,6-dichloro-4-trifluoromethylphenyl)-3-cyano-4-trifluoromethylsulfinyl-5-(ethoxyacetamido)pyrazole.

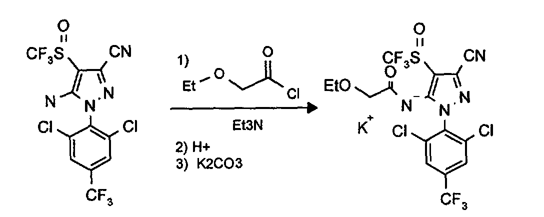

30g of ethoxyaecetyl chloride (0.233mol) was added to a mixture of 1-(2,6-dichloro-4-trifluoromethylphenyl)-3-cyano-4-trifluoromethylsulfinyl-5-amino-pyrazole (66g, 0.145mol) and triethylamine (44.5g, 0.435mol) in 100ml of tetrahydrofurane. The reaction mixture was stirred at 30°C during 5h, allowed to cool and 150mL of water and 150mL of CH2Cl2 were added. The pH was reduced to pH 2 with concentrated hydrochloric acid and the product extracted with CH2Cl2. A solution of potassium carbonate (50%) was added and the resulting precipitate concentrated to provide Compound II wherein W is potassium. (yield = 65%, assay = 77%).

Step 2: Preparation of 1-(2,6-dichloro-4-trifluoromethylphenyl)-3-cyano-4-trifluoromethylsulfinyl-5-(ethoxyacetamido- methyl)pyrazole. 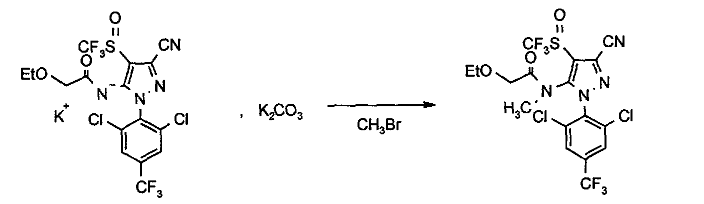 To a suspension of the potassium salt of 1-(2,6-dichloro-4-trifluoromethylphenyl)-3-cyano-4-trifluoromethylsulfinyl-5-(ethoxyacetamido)pyrazole, prepared in Step 1 above, (18.9g, assay =75.6%, 0.026mole) in 56.8g of acetonitrile, a solution of methyl bromide in acetonitrile (86.5g, conc =28%, 0.255mole) was added. The mixture was stirred during 6 hours at 60°C and then concentrated to dryness. The residue was solubilized in a mixture of toluene (100g) and water (100g). The organic layer was washed with 100g of water and concentrated to a 38% solution, heated to 80°C and product was recrystallized in a 40/60 toluene/n-heptane solution to afford 10.3g of a white solid (yield = 64%, assay =85 %).

### Example 2

Step 1: Preparation of the TEA salt of 1-(2,6-dichloro-4-trifluoromethylphenyl)-3-cyano-4-trifluoromethylsulfinyl-5-(ethoxyacetamido)pyrazole.

3.32g of ethoxyacetyl chloride (0.03mol) was added to a mixture of 1-(2,6-dichloro-4-trifluoromethylphenyl)-3-cyano-4-trifluoromethylsulfinyl-5-amino-pyrazole (8.74g, 0.02mol) and triethylamine (8.4ml, 0.06mol) in 20ml of tetrahydrofuran. The reaction mixture was stirred at 60°C during 1h and 1.1g (0.01mmol) of ethoxyacetyl chloride was added to the medium. After stirring for 30 minutes, the reaction mixture was allowed to cool and 20 ml of water and 20 ml of CH₂Cl₂ were added. The organic layer was washed with 10 ml of water and dried over magnesium sulphate. 12.5 g of Compound II, wherein W is triethylamine, was obtained giving a yield of 90% and an assay of 76%.

Step 2: 0.42 mole of the tri ethyl amine salt of 1-(2,6-dichloro-4-trifluoromethylphenyl)-3-cyano-4-trifluoromethylsulfinyl-5-(ethoxyacetamido)pyrazole, prepared according to step 1 above, was disscolved in 5ml of CH₂Cl₂. The pH was acidified to pH 2 with concentrated hydrochloric acid and the organic layer separated. The organic layer was then treated with a concentrated solution of NaOH (1.5 equivalents) and iodomethane (1.5 equivalents) to provide a yield of 40% of Compound I.

### Example 3

Step 1: 3.1g of Ethoxyacetyl chloride (0.024mol) was added during 2h to a mixture of 1-(2,6-dichloro-4-trifluoromethylphenyl)-3-cyano-4-trifluoromethylsulfinyl-5-amino-pyrazole (10g, 0.022mol) and KOH (3.2g, 0.57mol) in 7g of CH3CN. The reaction mixture was stirred at -5°C during 2h and the resulting mixture filtered : 15g of the wet solid was obtained. After drying 12.2g of compound II, wherein W is potassium, was obtained (yield = 87%, assay = 82%).

Step 2: Preparation of 1-(2,6-dichloro-4-trifluoromethylphenyl)-3-cyano-4-trifluoromethylsulfmyl-5-(ethoxyacetamido- methyl)pyrazole.

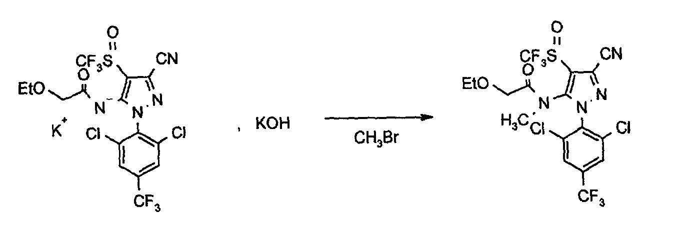

To a suspension of the potassium salt of 1-(2,6-dichloro-4-trifluoromethylphenyl)-3-cyano-4-trifluoromethylsulfinyl-5-(ethoxyacetamido)pyrazole (0.251 g, assay =82%, 0.36mmole) in 1.3g of acetonitrile, a solution of methyl bromide in acetonitrile (0.7g, conc =28%, 2.1mole) was added. The mixture was stirred during 6 hours at 60°C in a pressure vessel. Chemical Yield of the final compound is 85%.

## Claims

1. A process for the preparation of a compound of General Formula (I): 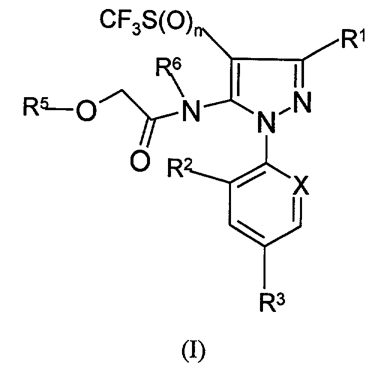 wherein:
R¹ is CN or CSNH₂;
X is N or CR⁴;
R² and R⁴ are each independently hydrogen or chlorine;
R³ is halogen, haloalkyl, haloalkoxy or -SF₅;
R⁵ and R⁶ are each independently an alkyl group; and
n is 0, 1 or 2;
which process comprises (a) a first step of reacting a compound of formula (II): 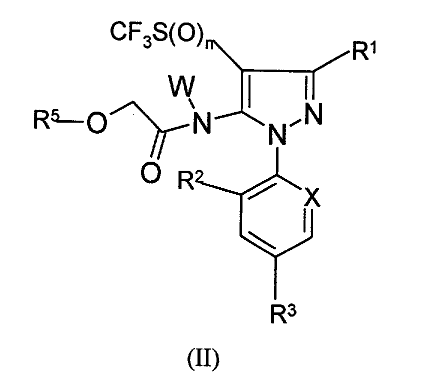 wherein the various symbols are as defined above and W is H, with an inorganic metal salt or an organic base to produce an intermediate compound, and (b) a second step of reacting the intermediate compound product with an alkylating agent of formula (III):
R⁶-Y (III)
wherein R⁶ is as defined above and Y is a leaving group.

2. A process as claimed in to claim 1 in which inorganic metal salt is a Group I or Group II metal salt selected from cesium, potassiun, sodium, magnesium and calcium.

3. A process as claimed in claim 1 or claim 2 in which the inorganic salts is a hydroxide, a carbonate or a hydrogen carbonate.

4. A process as claimed in any one of the preceding claims in which the inorganic salt is potassiun carbonate or potassium hydroxide.

5. A process as claimed in any one of the preceding claims in which the organic base is an amine selected from triethyl amine, pyridine and the like.

6. A process as claimed in any one of the preceding claims in which the compound of General Formula (II) is reacted with the metal salt or the organic base in a ratio of at least one equivalents, preferably 2 equivalents.

7. A process as claimed in any one of the preceding claims in which the alkylating agent is an alkyl halide, alkyl sulphonates or is an alkyl sulphate.

8. A process as claimed in claim 7 in which the alkylating agent is methyl bromide, methyl iodide or di methyl sulphate.

9. A process as claimed in any one of the preceding claims wherein steps 1 and 2 are carried out in the presence of a solvent.

10. A process as claimed in any one of the preceding claims wherein steps 1 and 2 are carried out in the presence of a base

11. A process as claimed in any one of the preceding claims in which the compound of General Formula II has representations R¹ is CN; X is CR⁴; R² and R⁴ are each chloride, R³ is trifluoromethyl; R⁵ is ethyl and W is an inorganic salt or an organic base; and n is 1

12. Novel compound as claimed as the intermediate compound as defined on any one of the preceding claims according to General Formula (II) wherein W is an inorganic salt or an organic base

13. Novel compound as claimed in claim 12 in which W is potassium.

14. A process for the preparation of a compound of formula (II) as defined in claim 1 or 2, which process comprises the reaction of a compound of formula (IV): 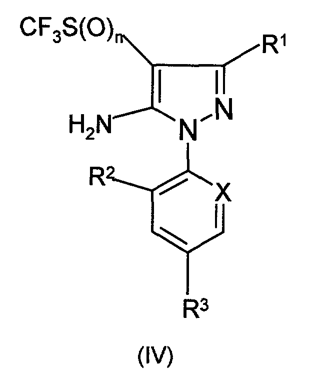 wherein the various symbols are as defined above, with an acylating agent of formula (V) or (V): 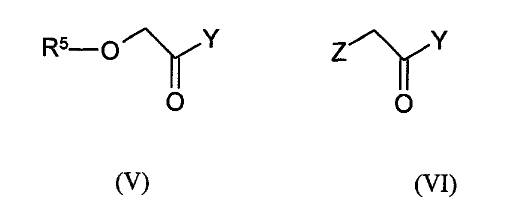 wherein R⁵ and Y are as defined above and Z is a halide, chloride, bromide or iodide.

15. A process as claimed in claim 14 wherein R⁵ and Y of compound (V) are ethyl and chloride respectively.

16. A process as claimed in claim 14 wherein Z and Y of compound (VI) are each chloride.

17. A process as claimed in any one of claims 14 to 16 carried out in the presence of solvent.

18. A process as claimed in any one of claims 11 to 17 carried out in the presence of an inorganic or organic base.
